# EUROPÄISCHE PATENTSCHRIFT

(11) **EP 3 381 376 B1**
(45) Veröffentlichungstag und Bekanntmachung des Hinweises auf die Patenterteilung: **01.05.2024**
(21) Anmeldenummer: 18164843.7
(22) Anmeldetag: 29.03.2018
(51) Int. Cl.: A61B 17/02, A61F 2/90, A61B 17/00, A61B 90/00

(54) **CHIRURGISCHES RETRAKTORSYSTEM MIT EINEM RETRAKTOR UND EINEM EXTRAKTOR**
SURGICAL RETRACTOR SYSTEM WITH A RETRACTOR AND AN EXTRACTOR
SYSTÈME RÉTRACTEUR CHIRURGICAL DOTÉ D'UN RÉTRACTEUR ET D'UN EXTRACTEUR

(30) Priorität: 30.03.2017 DE 102017106846
(43) Veröffentlichungstag der Anmeldung: 03.10.2018
(73) Patentinhaber: Aesculap AG, 78532 Tuttlingen (DE)
(72) Erfinder: BEGER, Jens, 78532 Tuttlingen (DE); SERPA, Mario, 78532 Tuttlingen (DE); STORZ, Frank-Markus, 78532 Tuttlingen (DE); KILIAN, Francis, 56281 Emmelshausen (DE)
(74) Vertreter: Winter, Brandl - Partnerschaft mbB

(56) Entgegenhaltungen:
- WO-A1-2016/007412
- DE-A1-102015 100 932
- US-A- 6 083 225
- US-A1- 2008 033 528
- US-A1- 2008 058 606
- US-A1- 2009 143 861
- US-A1- 2011 040 366
- US-A1- 2012 088 979
- US-A1- 2016 346 084
- US-B1- 6 582 431
- US-B2- 6 821 291

## Beschreibung

Die vorliegende Erfindung betrifft ein chirurgisches Retraktorsystem gemäß dem Oberbegriff des Patentanspruchs 1. Dieses weist einen Retraktor und einen Extraktor auf. Der Retraktor ist insbesondere stentartig ausgestaltet und zusätzlich dazu vorbereitet, eine Distraktor-Funktion zu erfüllen.

### Hintergrund der Erfindung

Ein chirurgisches Retraktorsystem mit einem Retraktor und einem Extraktor ist zum ersten dazu in der Lage, ein Operationsfeld / eine Inzision offenzuhalten. Hierfür wird der Retraktor von außerhalb eines zu behandelnden Patienten in das Operationsfeld eingesetzt und von dem Retraktor ausgebildete Spreizelemente / Aufweitungsabschnitte voneinander beabstandet. Dadurch wird Binde- und/oder Muskelgewebe auseinandergedrückt und somit das Operationsfeld erweitert. Zum zweiten ist der zumindest eine Extraktor des chirurgischen Retraktorsystems bzw. das chirurgische System dazu ausgelegt, die kraftbeaufschlagten Spreizelemente am Ende einer Operation oder eines Operationsschrittes wieder zu komprimieren, um ein Lösen des Retraktors aus dem Patienten zu ermöglichen.

Ein Distraktor hingegen ist allgemein ein chirurgisches Instrument bzw. Gerät zum kontrollierten Spreizen / Auseinanderziehen von Körperstrukturen durch mechanische Einwirkung von außen (Distraktion). Distraktoren werden etwa in der Unfallchirurgie, der Orthopädie und Kieferchirurgie zur Extensionsbehandlung von Knochen eingesetzt.

### Stand der Technik

Aus dem Stand der Technik sind Retraktoren bekannt, die neben der Retraktor- auch eine Distraktor-Funktion erfüllen. So offenbart eine weitere Anmeldung der Aesculap AG (DE 10 2015 100932 A1) einen Stent-Retraktor/Distraktor, also einen Retraktor mit einer Stentstruktur. Dieser weist einen radial flexibel aufweitbaren, rohrförmigen Mantel auf, der in Umfangsrichtung in wenigstens zwei Abschnitte, nämlich einen Aussteifungsabschnitt und einen Aufweitungsabschnitt mit zueinander unterschiedlicher radialer Flexibilität unterteilt ist, die stoffeinstückig miteinander verbunden sind. So ist eine Anpassung des Stent-Retraktors/Distraktors an eine patientenseitige Öffnung ermöglicht.

Als Extraktor, also als das Instrument zum Entfernen / Lösen des Stent-Retraktors/Distraktors aus der patientenseitigen Öffnung, wird in der vorstehend erwähnten Anmeldung eine Kompressionszange offenbart. Diese komprimiert den Stent-Retraktor/Distraktor so lange, bis dieser lösbar ist. Eine Kompressionszange ist in Operationssälen meist vorhanden und durchläuft nach jeder Benutzung einen aufwendigen Desinfektionszyklus.

Nachteilig an diesem Stand der Technik ist zum ersten, dass die zerstörerische Wirkung der Kompressionszange auf den Stent-Retraktor/Distraktor von Einzelfall zu Einzelfall variiert, woraus häufig ein zeitaufwendiger und unvorhersehbarer Lösevorgang des Retraktors aus der patientenseitigen Öffnung resultiert. Weiterhin zieht der Einsatz der Kompressionszange nach jeder Benutzung einen kosten- und zeitaufwendigen Desinfektionsprozess nach sich, was die Wirtschaftlichkeit des Retraktorsystems beeinträchtigt.

Nicht zuletzt sind im Stand der Technik die Voraussetzungen für ein intra-operatives, das heißt während des chirurgischen Eingriffs, Entfernen des Retraktors so ungünstig, dass zusätzliche Zeit sowie Instrumente einzuplanen sind, um einen planmäßigen Ablauf gewährleisten zu können.

US 2008/0033528 A1 beschreibt einen stentartigen Retraktor, dessen Radius über einen Extraktor mit distal angeordneten Greifelementen, die in Ösen am proximalen Ende des Retraktors eingreifen können, durch ein Bewegen der in die Ösen eingehakten Greifelemente aufeinander zu verengt werden kann.

US 2011/0040366 A1 offenbart einen stentartigen Retraktor, in dessen Struktur am proximalen und am distalen Ende ein Faden eingefügt werden kann, sodass der Retraktor im Zustand eines entspannten Fadens entfaltet ist und im Zustand eines gespannten Fadens einen verengten Radius aufweist.

US 6 821 291 B2 offenbart hingegen einen Stent, der einem Patienten zur Expansion eines Blutgefäßes oder ähnlichem implantiert wird. Zur Entnahme eines derartigen Stents ist an diesem eine Art Schnur vorgesehen, die über ein weiteres Element so um den Stent zusammengezogen werden kann, dass dieser aus dem Patienten (Blutgefäß oder ähnlichem) entfernbar ist.

US 2016/346084 A1 zeigt einen stentartigen Rahmen zur Verbesserung einer Herzklappe, dessen Radius über eine Drehbewegung einer Schraube, die in einem am Rahmen vorgesehenen Gewinde verschraubbar ist, verändert werden kann.

### Kurzbeschreibung der Erfindung

Angesichts des vorstehend genannten Stands der Technik ist es die Aufgabe der vorliegenden Erfindung, die Nachteile aus dem Stand der Technik zu beheben und insbesondere ein chirurgisches Retraktorsystem zu offenbaren, das ein effizientes Lösen / Entfernen des Retraktors, etwa aus der patientenseitigen Öffnung, ermöglicht. Hierbei steht vor allem das definierte Verformen des Retraktors durch den Extraktor im Mittelpunkt, das ein betriebssicheres sowie ein zeiteffizientes Lösen in Aussicht stellt.

Des Weiteren ist es ein bevorzugtes Ziel der vorliegenden Erfindung, den logistischen Aufwand, der aufzubringen ist, um das chirurgische Retraktorsystem betriebsbereit zu halten, zu senken. Darüber hinaus soll auch die Verletzungsgefahr, die von den verformten Retraktoren, die vorzugsweise aus Metallblechen gefertigt worden sind, ausgeht, minimiert werden.

Eine weitere Aufgabe der Erfindung liegt darin, einen Retraktor bzw. ein chirurgisches Instrument bereitzustellen, das günstig herstellbar und leicht zu desinfizieren ist und das in der Anwendung robust ist und eine Fehlbedienung nahezu ausschließt.

Die vorstehend genannten Aufgaben und Ziele werden erfindungsgemäß mit einem chirurgischen Retraktorsystem gemäß dem Patentanspruch 1 gelöst/erreicht. Vorteilhafte Weiterbildungen des chirurgischen Retraktorsystems sind Gegenstand der Unteransprüche.

Aus dieser erfindungsgemäßen Ausgestaltung des chirurgischen Retraktorsystems lassen sich somit beispielsweise folgende weitere Vorteile ableiten:
- Ein definiertes Zerstören des Einweg-/Einmal-Retraktors ermöglicht ein intra-operatives Entfernen des Retraktors ohne Komplikationen.
- Der Plattenabschnitt des Retraktors, der für die Zusammenwirkung mit dem Retraktor vorbereitet ist, ermöglicht einen sicheren Zugang für den Extraktor, wodurch die Sicherheit des Retraktorsystems erhöht ist.
- Der Extraktor ist auf den entsprechenden Plattenabschnitt abgestimmt ausgestaltet, was weniger Flexibilität in der Bedienung erfordert, wodurch günstigere Komponenten einsetzbar sind.
- Durch die günstigere Herstellung des Extraktors ist dessen Ausgestaltung als EinwegBauteil möglich, wodurch ein sogenanntes Single-Use-Konzept realisierbar ist.
- Das Retraktorsystem ist mittels des Single-Use-Konzepts auch an Orten / in Kliniken / in Gesundheitszentren einsetzbar, an denen keine wirtschaftlich sinnvollen Sterilisationsmöglichkeiten herrschen.
- Die Sterilisationsprozesskosten lassen sich aufgrund des Single-Use-Konzepts senken.
- Mittels des vordefinierten Angriffspunkts für den Extraktor am Plattenabschnitt des Retraktors lässt sich das Auftreten von Graten / Schnittkanten bei dem plastisch verformten Extraktor vermeiden, was nicht nur die Sicherheit des Patienten, sondern auch die des Operationspersonals erhöht.
- Über eine hinsichtlich des internen Kraftflusses effizient ausgestaltete Zusammenwirkung zwischen dem Griffabschnitt und dem Wirkabschnitt der Extraktoreinheit / des chirurgischen Instruments lässt sich der Retraktor unter geringem Kraftaufwand verbiegen / verformen.
- Über eine Schlitzanordnung im Wirkabschnitt ist ein Poka-Yoke-Prinzip realisiert, was einer falschen Bedienung des chirurgischen Instruments durch einen Anwender, wie einen Chirurgen, vorbeugt.

Der Gegenstand der Anmeldung ist demnach zum ersten ein chirurgisches Retraktorsystem mit einem im Wesentlichen rohrförmigen Retraktor, der in Axialrichtung bevorzugt mehrere Segmente aufweist. Diese sind dann voneinander irreversibel trennbar, um eine Längenanpassung des Retraktors vorzunehmen, wodurch der Retraktor in seiner Längsrichtung flexibel ausgestaltbar ist. Zumindest eines der Segmente ist weiter bevorzugt in Umfangsrichtung in zumindest einen Aufweitungsabschnitt / ein Spreizelement und einen Plattenabschnitt / Extraktoreingriffsabschnitt unterteilt. Diese beiden Abschnitte weisen dann zueinander eine unterschiedliche radiale Flexibilität auf und sind vorzugsweise stoffeinstückig miteinander verbunden, sodass eine plastische Radialaufweitung des Retraktors ermöglicht ist. Jene Radialaufweitung des Retraktors dient, wie eingangs erwähnt, einerseits dazu, das Operationsfeld offenzuhalten. Andererseits ist über sie auch die von dem erfindungsgemäßen Retraktor durchführbare Distraktion ggf. realisierbar. Das chirurgische Retraktorsystem weist ebenso zumindest einen Extraktor / Kompressionsinstrument / Kompressionswerkzeug / Kompressionsmechanismus auf, der einen Griffabschnitt und einen Wirkabschnitt mit einem Längsschlitz aufweist, der durch einen ersten Schenkel und einen zweiten Schenkel definiert ist und derart auf den Plattenabschnitt aufsetzbar ist, dass er diesen an seiner nach radial innen zeigenden Fläche und an seiner nach radial außen zeigenden Fläche quasi linienförmig umgreift, sodass der erste Schenkel von radial innen den Retraktor kontaktiert, während der zweite Schenkel den Retraktor von radial außen kontaktiert, sodass er eine der Radialaufweitung des Retraktors entgegengesetzte, plastische Radialverengung des Retraktors hervorruft. Diese Radialverengung ruft eine Zerstörung des Retraktors hervor und ermöglicht, dass dieser aus der Öffnung entfernbar ist.

Gemäß der Anmeldung ist der zumindest eine Extraktor dazu vorbereitet, über seine beiden Schenkel mit dem Retraktor, vorzugsweise mit dessen zumindest einem Plattenabschnitt, für einen Torsionsmomenteintrag zusammenzuwirken, um die Radialverengung des Retraktors zu erwirken. Zwischen dem retraktorseitigen Plattenabschnitt und dem Extraktor liegt somit eine Art Matrizen-Patrizen Beziehung vor. Wird demnach der Plattenabschnitt in seiner Geometrie abgewandelt, so ist auf diese Änderung auch der Extraktor anzupassen. Mittels jenes Zusammenspiels der beiden Komponenten des Retraktorsystems ist eine zeiteffiziente Deformation des Retraktors sichergestellt, was ein intra-operatives Entfernen des Retraktors ermöglicht.

In einer vorteilhaften Ausführungsform ist der Extraktor dazu vorbereitet, mit seinen beiden Schenkeln die plastische Radialverengung des Retraktors mittels eines auf den Retraktor wirkenden Torsionsmoments zu erzwingen. Das Torsionsmoment bewirkt zum ersten eine Art Aufwicklung des Retraktors auf den Extraktor, was eine zuverlässige Radial- / Durchmesserverengung nach sich zieht. Zum zweiten ist die erfindungsgemäße Torsionsverformung mittels eines Formschlusses zwischen dem Extraktor und dem Retraktor realisierbar. Da bei den jeweiligen Komponenten auf eine ausreichende Steifigkeit geachtet wird, ist ein ungewolltes Lösen des Formschlusses nicht möglich. Somit ist die Sicherheit des Retraktorsystems erhöht, da etwa ein Abrutschen des Werkzeugs, wie es bei Kompressionszangen aus dem Stand der Technik häufig vorkommt, ausgeschlossen ist.

Insbesondere wenn der Extraktor einen Griffabschnitt und einen Wirkabschnitt mit einem Schlitz aufweist, der derart auf den Plattenabschnitt des Retraktors aufsetzbar ist, dass er diesen an seiner nach radial innen zeigenden Fläche und an seiner nach radial außen zeigenden Fläche umgreift, ist die Zuverlässigkeit des Formschlusses erhöht.

In einer weiteren Ausführungsform ist ein zweiter Extraktor über seine beiden Schenkel mit einem zweiten Plattenabschnitt desselben Retraktors derart zusammenwirkt, dass zwei Extraktoren über ihre Schenkel eine Radialverengung des einen Retraktors hervorrufen. Der Einsatz eines zweiten Extraktors ermöglicht es, zweihändig eine komfortable Verformung hervorzurufen. Der zweite Extraktor ist vorzugsweise baugleich zum ersten Extraktor ausgestaltet. Somit werden die Produktionskosten gesenkt. Zudem erlaubt der zweite Extraktor die Realisierung des Extraktors mit einer einfachen Geometrie (da sinngemäß zwei Extraktoren mit einer simplen Geometrie den gleichen Effekt hervorrufen wie ein Extraktor mit einer komplexen Geometrie). So werden die Herstellungskosten weiter gesenkt, was es ermöglicht, neben dem Retraktor auch den Extraktor und somit das gesamte Retraktorsystem als Einweg- / Einmalprodukt gemäß dem Single-Use-Konzept auszubilden.

In einer vorteilhaften Ausführungsform ist das chirurgische Retraktorsystem dadurch gekennzeichnet, dass die beiden Extraktoren über ihre Schenkel mittels einer zueinander entgegengesetzten Drehbewegung ein Aufwickeln / Aufrollen / Verdrillen / Aufbiegen des Retraktors auf die Extraktoren erzwingen. So induziert die Beaufschlagung des Retraktors mit einer entgegengesetzten Drehbewegung ein Torsionsmoment, das in seinem Funktionsprinzip dem einer Sardinendose ähnlich ist.

Insbesondere vorteilhaft ist es, wenn in Axialrichtung entlang des Plattenabschnitts eines Segments des Retraktors eine Sollbruchstelle angeordnet ist, um ein Einknicken des Retraktors an der Sollbruchstelle bei Beaufschlagung des Torsionsmoments über die Schenkel des Extraktors hervorzurufen. So ist garantiert, dass der Retraktor einer vorbestimmten Knick-/Bruchlinie folgt. Alternativ umfasst das erfinderische Konzept auch den Gedanken, dass die Sollbruchstelle angewinkelt zur Axialrichtung verläuft, um eine fächerartige Radialverengung hervorzurufen. Eine solche Sollbruchstelle erhöht zum ersten die Betriebssicherheit, da bei den entsprechenden Bruchkanten auf eine runde Form geachtet ist, zum zweiten ermöglicht sie dem behandelnden Chirurgen eine komfortable Radialverengung. Das im Stand der Technik oft mühselige Zerren an dem Retraktor ist somit vermieden.

In einem weiteren Aspekt ist ein chirurgisches Instrument zum Lösen eines Distraktors oder auch eines Retraktors aus einer von dem Distraktor/Retraktor offengehaltenen, vorzugsweise patientenseitigen, Öffnung beansprucht. Das chirurgische Instrument, das auch als Extraktoreinheit bezeichnet werden kann, weist zumindest den eine Grifflängsachse definierenden Griffabschnitt und zumindest den eine Wirklängsachse definierenden Wirkabschnitt auf, die über einen Verbindungsabschnitt / ein Verbindungsglied miteinander gekoppelt sind. Der Verbindungsabschnitt ordnet den Griffabschnitt und den Wirkabschnitt hierbei derart zueinander an, dass die Grifflängsachse und die Wirklängsachse zueinander an- bzw. abgewinkelt verlaufen, vorzugsweise in einem Winkel zwischen 60° und 140°, besonders bevorzugt 90°. Der Winkel zwischen der Grifflängsachse und der Wirklängsachse wird hierbei von der Wirklängsachse ausgehend in Richtung der Grifflängsachse wandernd beschrieben. Bei einem Winkel von 60° definiert der Verbindungsabschnitt demnach einen spitzen Winkel.

Insbesondere dann, wenn der Griffabschnitt des chirurgischen Instruments über die gesamte Länge entlang der Grifflängsachse einen im Wesentlichen kreisförmigen und im Wesentlichen konstanten Querschnitt aufweist und der Wirkabschnitt über die gesamte Länge entlang der Wirklängsachse einen im Wesentlichen kreisförmigen und im Wesentlichen konstanten Querschnitt aufweist, lässt sich das Ziel der günstigen Herstellungskosten realisieren, wodurch ein Single-Use-Konzept realisierbar wird. Bevorzugt entspricht der Durchmesser des Griffabschnitts hierbei dem Durchmesser des Wirkabschnitts. Abweichungen von bis zu 10% der Durchmesser voneinander sind ebenso von dem erfinderischen Gedanken erfasst.

Das chirurgische Instrument weist an seinem Wirkabschnitt einen Längsschlitz auf, der entlang der Wirklängsachse verläuft und der den Wirkabschnitt in zwei umfangsgetrennte Schenkel aufteilt, die hinsichtlich ihrer Größe, das heißt ihrer im Querschnitt eingenommenen Fläche, vorzugsweise gleichgroß oder zumindest ähnlich groß ausgebildet sind. Die Schenkel sind hierbei sowohl in ihrer Länge, als auch in ihrer Querschnittsfläche im Wesentlichen gleich groß. Anders ausgedrückt verläuft der Längsschlitz in einer Ebene, in der auch die Wirklängsachse verläuft.

Sobald der erste Schenkel des Wirkabschnitts den Retraktor von radial außen / lateral kontaktiert und der andere, zweite Schenkel des Wirkabschnitts den Retraktor von radial innen / medial kontaktiert, ist ein effizientes, fehlerunanfälliges Aufsetzen der Extraktoreinheit auf den Retraktor gewährleistet.

Besonders bevorzugt setzt sich das chirurgische Instrument aus zwei baugleichen abgewinkelten Extraktoren zusammen, wodurch den Komfort sowie die Bediengenauigkeit erhöht wird, da die von dem chirurgischen Instrument hervorgerufene plastische Verformung des Retraktors von beiden Händen eines Chirurgen eingeleitet werden kann, wonach in der einzelnen Hand weniger Kraft aufzubringen ist. Somit ist zudem die Präzision des chirurgischen Instruments gesteigert.

Eine weitere vorteilhafte Ausführungsform zeichnet sich dadurch aus, dass der Verbindungsabschnitt hinsichtlich der Länge seines abgewinkelten Verlaufs, also dem Betrag der Länge des Verbindungsabschnitts, wenn man diesen "geradebiegen" würden, klein ist verglichen mit der Länge des Wirkabschnitts entlang der Wirklängsachse und der Länge des Griffabschnitt entlang der Grifflängsachse. So ist eine robuste Anordnung ohne überflüssigen

Bauteilabschnitt realisiert, da der Griff- und der Wirkabschnitt den Großteil des chirurgischen Instruments darstellen und etwa für Scharniere oder Gelenke kein Material und/oder Bauraum aufzubringen ist.

Bevorzugt liegt dem zumindest einen Extraktor oder dem chirurgischen Instrument als Rohling ein im Querschnitt bevorzugt runder Voll- oder Hohlprofil-Stab zugrunde, dessen Verbindungsabschnitt durch Umformen, insbesondere durch Biegen, des Stabs ausgebildet ist und dessen Wirkabschnitt zumindest einen durch einen spanenden Fertigungsschritt, wie Sägen oder Schleifen, oder auch durch einen Stanz- oder Laserschritt erzeugten Längsschlitz aufweist, der das distale Stabende im Bereich des Wirkabschnitts in zumindest zwei vorzugsweise gleichgroße Schenkel teilt, wodurch sich das chirurgische Instrument als Ein-Weg-Bauteil eignet. So ist eine simple Geometrie mit wirtschaftlich herstellbaren Komponenten realisiert, die in der Anschaffung mit so geringen Kosten verbunden sind, dass sie unter Vermeidung eines Desinfektionsprozesses als Wegwerfprodukt / Einwegprodukt / Single-Use-Produkt einsetzbar sind.

Insbesondere wenn der Aufweitungsabschnitt stentartig / in Stentstruktur ausgestaltet ist, um die plastische Verformung in Radialrichtung zu ermöglichen, folgen Vorteile in der Handhabung mit dem Retraktorsystem.

Weiterhin zielt eine vorteilhafte Ausführungsform darauf ab, den zumindest einen Extraktor als Einwegbauteil auszugestalten, um einen Desinfektionsprozess zu vermeiden.

In anderen Worten lässt sich die Erfindung derart beschreiben, dass zwei geschlitzte Extraktoren von außen / distal formschlüssig auf die Plattenabschnitte, die dem radialflexiblen Retraktor eine Steifigkeit verleihen, aufsetzbar sind. Nach dem Aufsetzen ist es möglich, die beiden Extraktoren gegeneinander so zu verdrehen, dass sie den Retraktor, der sich aufgrund des Formschlusses mit der Rotation des Extraktors mitverdreht, so verformen und in seinem Durchmesser so verkleinern, dass dieser leicht aus seiner Position lösbar ist.

Weiterhin ist es möglich, den zumindest einen Extraktor derart auf den Retraktor aufzusetzen, dass auf diesen auch eine Zugkraft übertragbar ist. Vorzugsweise ist hierbei die Zugkraft mit dem Torsionsmoment in Serie zu schalten, um zunächst mittels der Zugkraft aus einer annähernden Trichterform des Retraktors eine Rohrform herzustellen, bevor diese mittels des Torsionsmoments aufgerollt wird.

Gemäß einem weiteren Aspekt der vorliegenden Erfindung wird ein Retraktorsystem mit einem Retraktor-Stent, also ein Retraktor mit einer Stentstruktur, vorgeschlagen, der zur Verwendung als Retraktor angepasst ist. Durch die erfindungsgemäße Aufteilung der Wandstruktur in Abschnitte mit höherer und niedrigerer Flexibilität erhält der Retraktor-Stent zum einen die Eigenschaft, beliebig in Radialrichtung deformierbar zu sein (z.B. rund, oval, etc.), während er zeitgleich eine ausreichende Steifigkeit bewahrt, um Gewebe in auseinander gedrücktem Zustand zu halten. So kann ein minimal-invasiver Zugang beispielsweise in Form eines lumbalen, thorakalen, und/oder cervicalen Wirbelsäulenzugangs geschaffen werden. Auch sind craniale Anwendungen möglich.

Die Aufweitungsabschnitte dienen dafür, den Durchmesser des Retraktor-Stents verändern zu können, wohingegen die Plattenabschnitte / Versteifungsabschnitte die Standhaftigkeit des Stents gegen äußere radiale Kräfte zumindest in bestimmten Radialrichtungen erhöhen und zugleich eine definierte Anlagefläche für den Extraktor liefern. Dadurch ist es weiterhin möglich, den Retraktor-Stent alternativ oder zusätzlich zu seiner Retraktionsfunktion auch als Distraktor-Instrument einzusetzen, da seine insbesondere über die Versteifungsabschnitte prinzipiell erzielbaren Widerstandskräfte gegen radial wirkende Komprimierkräfte ausreichend sind, um auch Knochen auseinander zu halten.

Durch die vorzugweise stoffeinstückige Ausgestaltung des Retraktor-Stents ist eine einfache und schnelle Herstellungsart, beispielsweise durch Laser- oder Wasserschneiden von Metallblechen, möglich.

Weiter vorzugsweise ist die Wandstruktur (der Mantel) des Retraktor-Stents in Umfangsrichtung in vier Abschnitte unterteilt, von denen zwei mit im Wesentlichen gleicher Flexibilität als Plattenabschnitte / Versteifungsabschnitte ausgelegt sind und die zwei weiteren mit im Wesentlichen gleicher Flexibilität als Aufweitungsabschnitte ausgelegt sind, wofür die beiden Plattenabschnitte / Versteifungsabschnitte zumindest im Umfangsrichtung und vorzugsweise auch in Axialrichtung eine höhere Steifigkeit aufweisen als die beiden Aufweitungsabschnitte. Durch diese Weiterbildung ist der Retraktor-Stent mit einfachen Hilfsmitteln, wie beispielsweise einem Dilatationsballon oder einem Spekulum oder einem Langenbeckhaken, vorzugsweise gleichmäßig (symmetrisch) oder auch oval aufweitbar. Insbesondere zu diesem Zweck können die beiden Abschnitte mit jeweils gleicher Flexibilität auch diametral gegenüberliegend angeordnet sein.

Vorteilhaft ist es weiterhin, wenn die Segmente des Retraktor-Stents über axiale Verbindungselemente / Stege mit vorzugsweise höherer Steifigkeit miteinander gekoppelt sind, welche weiter vorzugsweise Sollbruchstellen zur segmentweisen Längenverkürzung bilden. Durch diese Ausgestaltung ist gewährleistet, dass sich der Retraktor-Stent bei innerer Druckbelastung im Wesentlichen nur radial aufweitet. Darüber hinaus ist der Stent entsprechend seinem beabsichtigten Einsatzort am Patientenkörper in seiner Länge einfach anpassbar.

Im Prinzip folgt die Funktionsweise des erfindungsgemäßen Extraktors demzufolge jener Funktionsweise einer Öffnungsvorrichtung einer allgemein bekannten Konservendose. Dort wird manchmal ein Aufwickelstab verwendet, der einen Längsschlitz aufweist, in welchen eine Lasche an einem abzuziehenden Dosendeckel eingeführt werden kann. Sobald der Aufwickelstab dann an einem daran ausgebildeten Griff um dessen Längsachse gedreht wird, wickelt sich der Dosendeckel auf den Aufwickelstab auf und gibt somit den Dosenzugang frei.

Die vorliegende Erfindung macht sich dieses Prinzip zunutze, indem der erfindungsgemäße Extraktor wenigstens einen solchen Aufwickelstab als Extraktor mit daran ausgebildetem Griff(-abschnitt) oder Hebel hat, der mit dem rohr-/hülsenförmigen Retraktor/Stent bzw. dessen Wandung derart in (linienförmigen) Formschluss bringbar ist (erfindungsgemäß über einen Längsschlitz am Aufwickelstab oder ansonsten über einen radial vorragenden Mitnahmebolzen), um ein über den Hebel auf den Aufwickelstab eingetragenes Drehmoment um dessen Längsachse auf die Wandung des Retraktors/Stents zu übertragen und so ein Einknicken und Aufwickeln der Wandung in Retraktor-Längsrichtung zu bewirken.

Wenn zwei solcher Aufwickelstäbe/Extraktoren bevorzugt vorgesehen sind, können diese beispielsweise an diametral sich gegenüberliegenden Winkelpositionen an der Wandung des Retraktors/Stents formschlüssig eingreifen und ein symmetrisches Aufwickeln der Wandung an den entsprechend beiden Positionen bewirken.

Sofern die beiden Aufwickelstäbe (mittig) miteinander scharnierartig sowie relativ um deren Längsachse frei drehbar gekoppelt sind (um so eine Extraktoreinheit auszubilden), können die Aufwickelstäbe auch an deren Griffen/Hebeln scherenartig auseinander gespreizt werden, sodass der Retraktor/Stent mittels der erfindungsgemäßen Extraktoreinheit radial aufgeweitet werden kann.

Die Erfindung wird nachstehend anhand bevorzugter Ausführungsbeispiele unter Bezugnahme auf die begleitenden Figuren näher erläutert. Die Figuren sind lediglich schematischer Natur und dienen ausschließlich dem Verständnis der Erfindung. Die gleichen Elemente sind mit denselben Bezugszeichen versehen. Es zeigen:
- Fig. 1:: eine schematische Ansicht eines erfindungsgemäßen Retraktorsystems in einer ersten Ausführungsform, bei der ein Extraktor mit einem Plattenabschnitt zusammenwirkt;
- Fig. 2:: zwei erfindungsgemäße Extraktoren in einer ersten Ausführungsform;
- Fig. 3a, b:: das Retraktorsystem, bei dem ein Retraktor von den beiden Extraktoren auf Torsion belastet ist;
- Fig. 4:: einen von dem Extraktor plastisch verformten Retraktor alleine dargestellt; und
- Fig. 5:: einen Retraktor in einer weiteren Ausführungsform mit einer Sollbruchstelle.

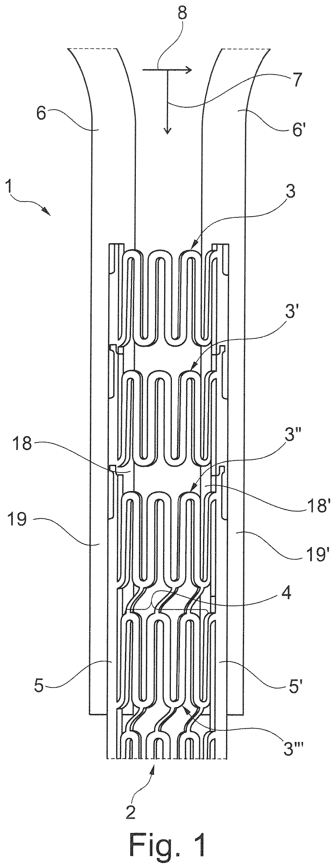

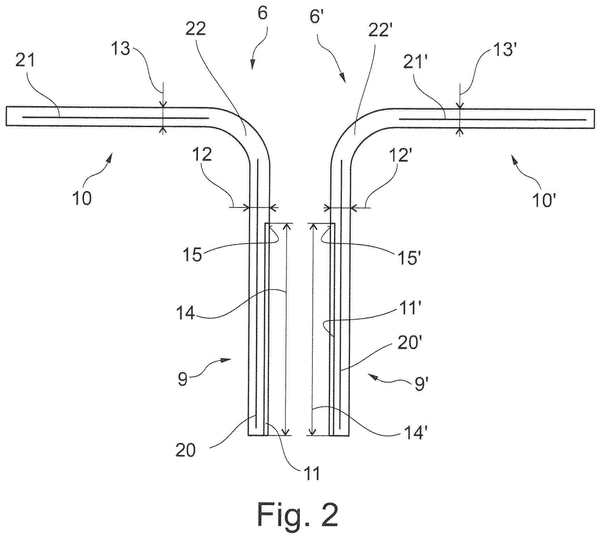

Fig. 1 zeigt ein chirurgisches Retraktorsystem 1 mit einem Retraktor 2 und zwei Extraktoren 6, 6', die auch als eine Extraktoreinheit 6, 6' oder allgemein als chirurgisches Instrument angesehen werden können. Von den Extraktoren 6, 6' ist nur ein Wirkabschnitt 9, nämlich der Aufwickelstab, dargestellt. Bezüglich eines Griffabschnitts 10 der Extraktoren 6, 6' sei auf Fig. 2 verwiesen.

Der Retraktor 2 ist in der Längsrichtung / Axialrichtung, die mittels eines Pfeils 7 angedeutet ist, in mehrere Segmente 3, 3', 3", 3‴ aufgeteilt. Die jeweiligen Segmente 3, 3', 3", 3‴ sind in ihrer Struktur gleichartig ausgestaltet. Direkt nach einem Einsetzen des Retraktors 2 in eine patientenseitige Öffnung ermöglicht die segmentartige Ausgestaltung in Axialrichtung ein Abtrennen einzelner Segmente 3, 3', 3", 3‴ voneinander, wodurch eine Längenanpassung des Retraktors 2 realisierbar ist.

In Umfangsrichtung, die in Fig. 1 mittels des Pfeils 8 angedeutet ist, ist der Retraktor 2 pro Segment 3, 3', 3", 3‴ in jeweils zwei Aufweitungsabschnitte / Spreizabschnitte 4, von denen in der Ansicht in Fig. 1 nur einer sichtbar ist, und zwei Plattenabschnitte 5, 5' unterteilbar. Der Aufweitungsabschnitt 4 und der Plattenabschnitt 5, 5' weisen eine unterschiedliche radiale Flexibilität auf. So ist der Aufweitungsabschnitt 4 mittels einer stentartigen Struktur, die im vorliegenden Beispiel über eine Wellenform mit einer verglichen mit ihrer Frequenz hohen Amplitude ausgebildet ist, plastisch verformbar, was eine Radialaufweitung des Durchmessers des rohrförmigen Retraktors 2 ermöglicht. Die Aufweitungsabschnitte 4 und die Plattenabschnitte 5, 5' sind zueinander diametral gegenüberliegend angeordnet, woraus eine wechselseitige Anordnung der zueinander unterschiedlichen Abschnitte resultiert.

Neben der radialen Flexibilität, die im Wesentlichen von den Aufweitungsabschnitten 4 gewährleistet wird, sorgt das erfindungsgemäße Retraktorsystem 1 für eine ausreichende Robustheit des Retraktors 2. Diese ist einerseits benötigt, um die eingangs erwähnte Distraktorfunktion durch den Retraktor 2 zu realisieren und andererseits um einen Ansatzpunkt / eine Koppelstelle für die Extraktoren 6, 6' auszuformen.

Die Exktraktoren 6, 6' sind Werkzeuge / Instrumente / ein chirurgisches Instrument vornehmlich zum Entfernen / Lösen des Retraktors 2 aus einer patientenseitigen Öffnung. Sie nehmen bei Betätigung durch einen Chirurgen mittels einer plastischen Verformung des Retraktors 2 eine Radialverengung vor, die das vom Retraktor 2 zuvor hervorgerufene Offenhalten / Aufspreizen beendet und ein intra-operatives Entfernen des Retraktors 2 ermöglicht. Die Extraktoren 6, 6' werden mithilfe eines im Zusammenhang mit Fig. 2 näher vorgestellten Längsschlitzes 11, 11' derart auf den zu lösenden Retraktor 2 aufgesetzt, dass ein erster Schenkel 18, 18', der wiederum in Fig. 1 gut erkennbar ist, von radial innen / medial den Retraktor 2 kontaktiert, während ein zweiter Schenkel 19, 19' den Retraktor von radial außen / lateral kontaktiert. Mittels dieser beiden Schenkel 18, 18', 19, 19' ist die erfindungsgemäße Aufwickelfunktion des Aufwickelstabs / Wirkabschnitts 9, 9` realisierbar.

Um die plastische Verformung durch die Extraktoren 6, 6' zuverlässig und vorhersehbar zu gestatten, ist eine vordefinierte (Ein-)Wirkfläche der Extraktoren 6, 6' an dem Retraktor 2 Teil des erfinderischen Gedankens. Diese Wirkfläche wird von den Plattenabschnitten 5, 5' gewährleistet, die eine ausreichende Steifigkeit aufweisen, um etwa eine Torsionsbelastung, die in diesem Ausführungsbeispiel von den Extraktoren 6, 6' auf den Retraktor 2 bzw. dessen Wand eingeleitet wird, derart auf die Aufweitungsabschnitte 4 weiterzugeben, dass sich der Retraktor 2 plastisch radialverengt.

In Fig. 2 sind die Extraktoren 6, 6' aus Fig. 1 für sich alleine dargestellt. Der zuvor erwähnte Wirkabschnitt/Aufwickelstab 9 des Extraktors 6, bzw. der Wirkabschnitt/Aufwickelstab 9` des Extraktors 6`, ist um ca. 90° von dem Griffabschnitt 10, 10' abgewinkelt, der vorliegend einen Hebel bildet. Die 90° beziehen sich hierbei auf den Winkel zwischen einer Wirklängsachse 20, 20', die die Längsachse des Wirkabschnitts 9, 9' darstellt, und einer Grifflängsachse 21, 21', die analog hierzu die Längsachse des Griffabschnitts 10, 10' darstellt. Die dargestellten 90° sind hierbei nicht einschränkend zu verstehen. Vielmehr sind erfindungsgemäß Winkelbereiche zwischen 60° und 140° erreichbar. Der Zwischenabschnitt zwischen dem Wirkabschnitt 9, 9' und dem Griffabschnitt 10, 10' ist von einem Verbindungsabschnitt 22, 22' realisiert. Der Verbindungsabschnitt 22, 22' ist bevorzugt mittels eines Umformverfahrens realisiert. So ist das chirurgische Instrument / der Extraktor 6 im Rohzustand ein Stab mit einheitlichem Durchmesser, der zunächst im Bereich des Verbindungsabschnitts 22, 22' um die gewünschte Winkelspanne umgeformt wird, bevor oder alternativ nachdem der Längsschlitz etwa spanend in den Wirkabschnitt 9, 9' eingebracht wird / wurde. Der Verbindungsabschnitt 22, 22` ist hinsichtlich seiner Länge entlang der bei ihm gebogenen Längsachse klein vergleichen mit dem Wirkabschnitt 9, 9` und mit dem Griffabschnitt 10, 10'.

Der Längsschlitz 11, 11' verläuft vorzugsweise durch den Querschnittsmittelpunkt des Wirkabschnitts 9, 9`. Der Schlitz 11, 11' ist derart dimensioniert, dass er über / auf den Plattenabschnitt 5, 5' in Längsrichtung des Retraktors/Stents stülpbar / aufsetzbar ist. D.h. die Schlitzweite am Extraktor 6, 6'erlaubt ein Einführen der Retraktorwandung in Retraktorlängsrichtung 7. Die Retraktorwandung setzt sich in Umfangsrichtung bevorzugt aus dem zumindest einen Aufweitungsabschnitt 4 und dem zumindest einen Plattenabschnitt 5, 5' zusammen. So entsteht in der Umfangsrichtung 8 des Extraktors ein Formschluss zwischen dem Retraktor 2 und dem Extraktor 6, 6', welcher wiederum bei einer Rotation des Extraktors 6, 6'um seine Längsachse ein Abknicken und Aufwickeln der Retraktorwandung (bei einem großen Übersetzungsverhältnis/Hebelwirkung) bewirkt, was die Radialverengung effizient ermöglicht. Jener Formschluss wird über die beiden im Zusammenhang mit Fig. 1 vorgestellten Schenkel 18, 19 ermöglicht, die den Schlitz 11 definieren.

Der Durchmessers 12, 12' des Wirkabschnitts 9, 9' entspricht vorzugsweise im Wesentlichen dem des Durchmessers 13, 13' Griffabschnitts 10, 10' und ist gegenüber dem Durchmesser des Retraktors/Stents deutlich kleiner. Die Einheitlichkeit des Durchmessers ist eine Folge daraus, dass der Extraktor 6 im Rohzustand, wie vorstehend beschrieben, bevorzugt ein Stab ist. Der Griffabschnitt 10, 10' weist überdies keine ergonomischen Besonderheiten auf. Somit ist der Extraktor 6, 6' ein wirtschaftlich herstellbares, unkompliziertes Stab-Bauteil, was es dem chirurgischen Retraktorsystem 1 ermöglicht, ein Single-Use-Konzept zu realisieren.

Eine Schlitzlänge 14, 14' des Schlitzes 11, 11' ist dazu vorbereitet, die Länge in Axialrichtung 7 von zumindest drei Segmenten 3, 3', 3" zu übersteigen. Auf diese Weise ist eine ausreichende Kraft- und Momentenübertragungsmöglichkeit des Extraktors 6, 6' auf den Retraktor 2 bzw. dessen Wandung sichergestellt. Ein Schlitzgrund 15, 15' des Schlitzes 11, 11' ist derart beschaffen, dass er einen Anschlag für die eine (proximale) Stirnseite des Retraktors 2 darstellt. So ist das axiale Aufsetzen des Extraktors 6, 6' auf den Retraktor 2 bzw. dessen Wandung zum einen über die Schlitzbreite, die mit dem Plattenabschnitt 4 zusammenwirkt und zum anderen über den Schlitzgrund 15, 15' vordefiniert realisierbar.

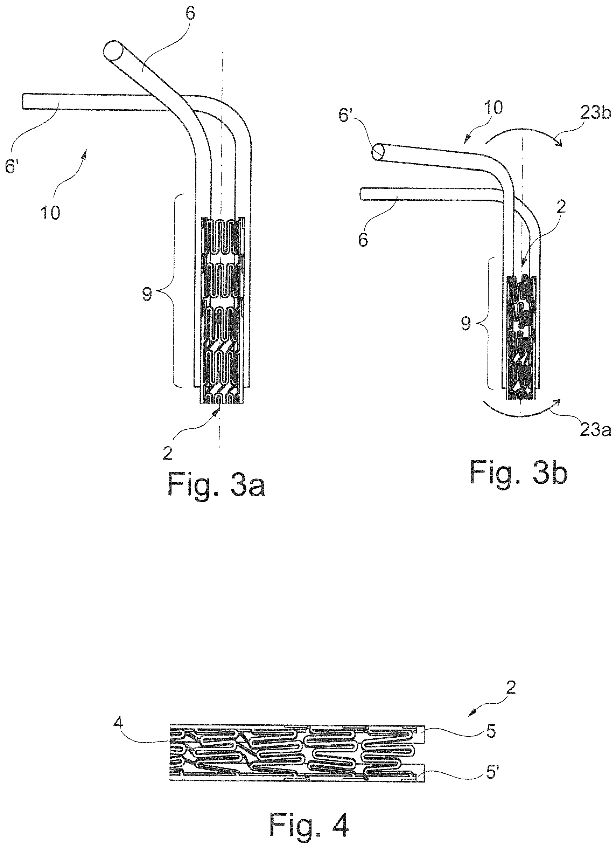

In den Figuren 3a und 3b ist die Radialverengung des Retraktors 2 durch die Extraktoren 6, 6' dargestellt. In den beiden Figuren hat eine Relativrotation der beiden Extraktoren 6, 6' zueinander um deren jeweilige Längsachsen stattgefunden, was eine plastische Verformung in Form einer Radialverengung des Retraktors zur Folge hat. Der Retraktor 2 ist über die Extraktoren 6, 6' mittels eines Torsionsmoments beaufschlagt worden, was zu einem Aufwickeln / Aufrollen / Verdrillen / "Crumblen" des Retraktors 2 bzw. dessen Wandung führt. Das Torsionsmoment ist über die beiden Pfeile 23a und 23b angedeutet, die eine gegenläufige Rotation und somit eine Torsion um die Längsachse des Retraktors 2 realisieren, was aus perspektivischen Gründen nicht unzweifelhaft aus der schematischen Abbildung in Fig. 3b hervorgeht. Während der distale Abschnitt des Retraktors 2, etwa über seine Verankerung in der patientenseitigen Öffnung, ein Moment in die erste Richtung erfährt (23a), wird der proximale Abschnitt des Retraktors durch den Extraktor 6, 6' in die zweite, andere Richtung momentbeaufschlagt (23b). Dies führt zu der erfindungsgemäßen Radialverengung des Retraktors 2.

In Fig. 4 ist der Retraktor 2 im radialverengten Zustand dargestellt. So ist er von den Extraktoren in Fig. 3a, 3b derart plastisch verformt worden, dass die Plattenabschnitte 5, 5' aneinander anliegen. Die Aufweitungsabschnitte 4 haben sich ebenfalls verformt. Der in Fig. 4 dargestellte eingerollte/aufgewickelte Retraktor ist leicht aus einer patientenseitigen Öffnung herausnehmbar.

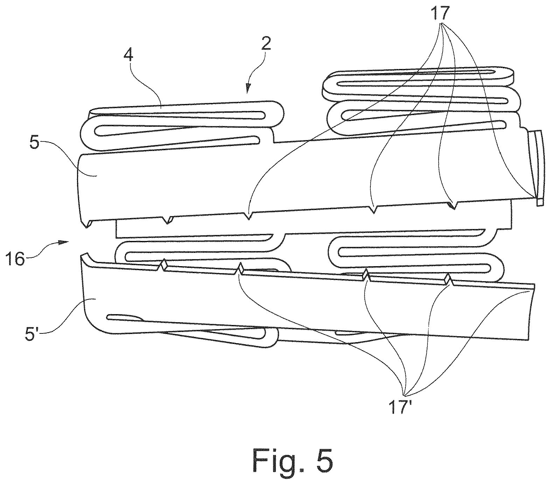

Fig. 5 zeigt einen Retraktor 2 in einer weiteren Ausführungsform. Zwischen den Plattenabschnitten 5, 5' ist eine Sollbruchstelle 16 vorgesehen. Diese ist in dem in Fig. 5 dargestellten Zustand bereits durchtrennt worden. Die Sollbruchstelle 16 ist über einzelne Vorsprünge 17, 17', die von dem jeweiligen Plattenabschnitt 5, 5' ausgeformt sind, realisiert. In dem nicht dargestellten Zustand, in dem die Sollbruchstelle 16 noch nicht durchtrennt ist, sind die Vorsprünge 17, 17', die auf der gleichen Höhe angeordnet sind, jeweils miteinander stoffschlüssig in Kontakt. Der Retraktor aus Fig. 5 ist bereits von hier nicht dargestellten Extraktoren 6, 6' verformt worden, was an der durchtrennten Sollbruchstelle 16 erkennbar ist.

Im Folgenden sei vor allem unter Bezugnahme auf Fig. 1 nochmals die Struktur des Retrakors 2 erläutert. Dessen Wandstruktur besteht in Umfangsrichtung gesehen aus zwei Umfangsabschnitten mit geringer Steifigkeit 4 und zwei Umfangsabschnitten mit vergleichsweise hoher Steifigkeit 5, 5', die in Umfangsrichtung wechselweise zueinander und in Achsrichtung 7 linear angeordnet sind.

Prinzipiell hat der erfindungsgemäße Retraktor 2 eine Rohr- oder Schlauchform, wobei die Umfangsabschnitte mit zueinander gleichen/ähnlichen Steifigkeiten einander diametral gegenüberliegen. Der Retraktor 2 ist ferner stoffeinstückig ausgebildet, d.h. die einzelnen Umfangsabschnitte sind stoffeinstückig miteinander verbunden.

Der Retraktor 2 hat im Wesentlichen eine Wandstruktur, die von einem Standard Gefäßstent (wie dem Coroflex der Aesculap AG) in Grundzügen übernommen ist. Das bedeutet, dass der Retraktor 2 zumindest im Bereich seiner Aufweitungsabschnitte (Aufweitungselemente) 4 aus einer Anzahl von axial beabstandeten, vorzugsweise parallel verlaufenden Bändern gebildet ist, die sich schlangen- oder ziehharmonikaförmig in Umfangsrichtung erstecken und somit im Bereich ihrer Ziehharmonikaform flexible Aufweitungsreserven in Radialrichtung bilden.

Zur Erhöhung der Stabilität sind die steiferen Umgangsabschnitte (Versteifungsabschnitte / Plattenabschnitte) 5, 5' in Umfangsrichtung gesehen jeweils zwischen den beiden Aufweitungsabschnitten 4 (wechselweise) angeordnet. Die Versteifungsabschnitte (Versteifungselemente) 5, 5' werden durch im Wesentlichen geschlossene, vorzugsweise rechteckige Plattenabschnitte ausgebildet, die in ihrer Grundform in Achsrichtung gesehen wannen- oder trogartig gewölbt und dafür vorgesehen sind, sich nicht oder nur geringfügig radial aufzuweiten. Die Versteifungsabschnitte 5, 5' werden auch dazu genutzt, um einen Angriffspunkt für den Extraktor 6, 6' auszubilden. Ziel ist es hierbei, möglichst viele Funktionalitäten in die Geometrie der Wandstruktur zu legen.

Als Werkstoff für den erfindungsgemäßen Retraktor 2 kann Stahl, Titan oder Kunststoff verwendet werden, wobei ein Kunststoffteil vorzugsweise im Spritzgussverfahren hergestellt wird. Des Weiteren kann der Retraktor 2 nach dem Schneiden der Wandprofile beispielsweise durch Elektropolieren entgratet werden. Um ferner die lichttechnischen Reflektionseigenschaften beispielsweise unter Mikroskopanwendungen zu verbessern, kann zudem die Oberfläche der verbleibenden Strukturen auch mattiert oder beschichtet werden.

## Patentansprüche

1. Chirurgisches Retraktorsystem (1) mit einem im Wesentlichen rohrförmigen Retraktor (2), der in Axialrichtung (7) eine Anzahl von Segmenten (3, 3', 3", 3‴) aufweist, wobei zumindest ein Segment (3) in Umfangsrichtung in zumindest einen Aufweitungsabschnitt (4) und einen Plattenabschnitt (5, 5') unterteilt ist, die eine zueinander unterschiedliche radiale Flexibilität aufweisen und stoffeinstückig miteinander verbunden sind, sodass eine plastische Radialaufweitung des Retraktors (2) ermöglicht ist, wobei
das chirurgische Retraktorsystem (1) zumindest einen Extraktor (6, 6') aufweist, der einen Griffabschnitt (10, 10') und einen Wirkabschnitt (9, 9') mit einem Längsschlitz (11, 11') aufweist, der durch einen ersten Schenkel (18, 18') und einen zweiten Schenkel (19, 19') definiert ist und derart auf den Plattenabschnitt (5, 5') aufsetzbar ist, dass er diesen an seiner nach radial innen zeigenden Fläche und an seiner nach radial außen zeigenden Fläche quasi linienförmig umgreift, sodass der erste Schenkel (18, 18') von radial innen den Retraktor (2) kontaktiert, während der zweite Schenkel (19, 19') den Retraktor (2) von radial außen kontaktiert, sodass im Falle einer Radialaufweitung eine der Radialaufweitung des Retraktors (2) entgegengesetzte, plastische Radialverengung des Retraktors (2) hervorgerufen wird, indem der zumindest eine Extraktor (6) über seine beiden Schenkel (18, 18', 19, 19') mit dem zumindest einen Plattenabschnitt (5) für einen Torsionsmomenteintrag zusammenwirkt, um dadurch die Radialverengung des Retraktors (2) zu erwirken.

2. Chirurgisches Retraktorsystem (1) nach Anspruch 1, **dadurch gekennzeichnet, dass** der Extraktor (6) mit seinen beiden Schenkeln (18, 18', 19, 19') die plastische Radialverengung des Retraktors (2) mittels eines solchen auf den Retraktor (2) wirkenden Torsionsmoments (22a, 22b) erzwingt, sodass ein Einknicken der Retraktorwandung in Retraktorlängsrichtung bewirkt ist.

3. Chirurgisches Retraktorsystem (1) nach Anspruch 1 oder Anspruch 2, **dadurch gekennzeichnet, dass** ein zweiter Extraktor (6') über seine beiden Schenkel (18, 18', 19, 19') mit einem zweiten Plattenabschnitt (5') desselben Retraktors (2) zusammenwirkt, sodass zwei Extraktoren (6, 6') über ihre Schenkel (18, 18', 19, 19') eine Radialverengung des einen Retraktors (2) hervorrufen.

4. Chirurgisches Retraktorsystem (1) nach Anspruch 3, **dadurch gekennzeichnet, dass** die beiden Extraktoren (6, 6') über ihre beiden Schenkel (18, 18', 19, 19') mittels einer zueinander entgegengesetzten Drehbewegung ein Aufwickeln des Retraktors (2) auf die Extraktoren (6, 6'), insbesondere deren Wirkabschnitte, erzwingen.

5. Chirurgisches Retraktorsystem (1) nach einem der Ansprüche 1 bis 4, **dadurch gekennzeichnet, dass** in Axialrichtung (7) entlang des Plattenabschnitts (5, 5') eines Segments (3, 3', 3", 3‴) des Retraktors (2) eine Sollbruchstelle (16) angeordnet ist, um ein Einknicken des Retraktors (2) an der Sollbruchstelle (16) bei Beaufschlagung des Torsionsmoments (22a, 22b) über die beiden Schenkel (18, 18', 19, 19') des Extraktors (6, 6') hervorzurufen.

6. Chirurgisches Retraktorsystem (1) nach einem der vorstehenden Ansprüche, **dadurch gekennzeichnet, dass** zumindest der Aufweitungsabschnitt (4) stentartig ausgestaltet ist, um die plastische Verformung zu ermöglichen.

7. Chirurgisches Retraktorsystem (1) nach einem der vorstehenden Ansprüche, **dadurch gekennzeichnet, dass** dem zumindest einen Extraktor (6) als Rohling ein im Querschnitt bevorzugt runder Voll- oder Hohlprofil-Stab zugrundeliegt, dessen Verbindungsabschnitt durch Umformen, insbesondere Biegen, des Stabs ausgebildet ist und dessen Wirkabschnitt zumindest einen durch einen spanenden Fertigungsschritt, wie Sägen oder Schleifen, oder durch einen Stanz- oder Laserschritt erzeugten Längsschlitz aufweist, der das distale Stabende im Bereich des Wirkabschnitts in zumindest zwei vorzugsweise gleichgroße Schenkel teilt, wodurch sich das chirurgische Instrument als Ein-Weg-Bauteil eignet.

8. Chirurgisches Retraktorsystem (1) nach einem der vorstehenden Ansprüche, **dadurch gekennzeichnet, dass** der Extraktor (6, 6') zum Lösen eines Distraktors oder Retraktors (2) aus einer von dem Distraktor/Retraktor (2) offengehaltenen, vorzugsweise patientenseitigen Öffnung, mit zumindest dem eine Grifflängsachse (21, 21') definierenden Griffabschnitt (10, 10') und zumindest dem eine Wirklängsachse (20, 20') definierenden Wirkabschnitt (9, 9'), die über einen Verbindungsabschnitt (22) miteinander gekoppelt sind, vorgesehen ist; und der Verbindungsabschnitt (22) den Griffabschnitt (21, 21') und den Wirkabschnitt (9, 9') derart zueinander anordnet, dass die Grifflängsachse (21, 21') und die Wirklängsachse (20, 20') zueinander abgewinkelt verlaufen, vorzugsweise in einem Winkel zwischen 60° und 140°, besonders bevorzugt 90°, wobei der Wirkabschnitt (9, 9`) den an seinem distalen Ende offenen Längsschlitz (11, 11') aufweist, der entlang der Wirklängsachse (20, 20') verläuft und den Wirkabschnitt (9, 9') in die zwei gleichgroßen, umfangsgetrennten Schenkel (18, 18', 19, 19') aufteilt.

9. Chirurgisches Retraktorsystem nach Anspruch 8, **dadurch gekennzeichnet, dass** der Griffabschnitt (10, 10') über die gesamte Länge entlang der Grifflängsachse (21, 21') einen im Wesentlichen kreisförmigen und einen im Wesentlichen konstanten Querschnitt aufweist und dass der Wirkabschnitt über die gesamte Länge entlang der Wirklängsachse (20, 20`) ebenfalls einen im Wesentlichen kreisförmigen und im Wesentlichen konstanten Querschnitt aufweist.

10. Chirurgisches Retraktorsystem nach Anspruch 9, **dadurch gekennzeichnet, dass** der Durchmesser (13, 13') des Griffabschnitts (10, 10') dem Durchmesser (12, 12') des Wirkabschnitts (9, 9') entspricht.

11. Chirurgisches Retraktorsystem nach einem der Ansprüche 8 bis 10, **dadurch gekennzeichnet, dass** der erste Schenkel (18, 18') des Wirkabschnitts (9, 9') dazu vorbereitet und so angeordnet ist, den Retraktor (2) von radial innen zu kontaktieren, und der zweite Schenkel (19, 19') des Wirkabschnitts (9, 9') dazu vorbereitet und so angeordnet ist, den Retraktor von radial außen zu kontaktieren.

12. Chirurgisches Retraktorsystem nach einem der Ansprüche 1 bis 11, **dadurch gekennzeichnet, dass** sich das chirurgische Retraktorsystem aus zwei baugleichen abgewinkelten Extraktoren (6, 6') zusammensetzt.

13. Chirurgisches Retraktorsystem nach einem der Ansprüche 1 bis 12, **dadurch gekennzeichnet, dass** der Verbindungsabschnitt (22, 22') hinsichtlich der Länge seines abgewinkelten Verlaufs klein ist verglichen mit der Länge des Wirkabschnitts (9, 9') und der Länge des Griffabschnitts (10, 10').

14. Chirurgisches Retraktorsystem (1) nach einem der Ansprüche 1 bis 13, **dadurch gekennzeichnet, dass** dem Retraktorsystem (1) als Rohling ein im Querschnitt bevorzugt runder Voll- oder Hohlprofil-Stab zugrundeliegt, dessen Verbindungsabschnitt durch Umformen, insbesondere Biegen, des Stabs ausgebildet ist und dessen Wirkabschnitt zumindest einen durch einen spanenden Fertigungsschritt, wie Sägen oder Schleifen, oder durch einen Stanz- oder Laserschritt erzeugten Längsschlitz aufweist, der das distale Stabende im Bereich des Wirkabschnitts in zumindest zwei vorzugsweise gleichgroße Schenkel teilt, wodurch sich das chirurgische Retraktorsystem als Ein-Weg-Bauteil eignet.

15. Chirurgisches Retraktorsystem nach Anspruch 14, wenn abhängig von Anspruch 12, **dadurch gekennzeichnet, dass** die zwei Extraktoren (6, 6') dafür vorgesehen und/oder aneinander derart gehalten sind, um zueinander gegenläufige Torsionsmomente gleichzeitig in den Distraktor/Retraktor (2) einzutragen.

## Claims

1. A surgical retractor system (1) comprising a substantially tubular retractor (2) having in axial direction (7) a number of segments (3, 3', 3", 3‴), wherein at least one segment (3) is subdivided in the circumferential direction into at least one expanding section (4) and one plate section (5, 5') which are of differing radial flexibility and connected to each other in a one-piece material bond so that a plastic radial expansion of the retractor (2) is made possible, wherein
the surgical retractor system (1) has at least one extractor (6, 6'), which has a handle section (10, 10') and an operating section (9, 9') with a longitudinal slit (11, 11'), which is defined by a first leg (18, 18') and a second leg (19, 19') and can be placed on the plate section (5, 5') in such a way that it engages around the latter in a quasi-linear manner on its radially inward-facing surface and on its radially outward-facing surface, so that the first leg (18, 18') contacts the retractor (2) from radially inside, while the second leg (19, 19') contacts the retractor (2) from radially outside, so that it brings about, in case of a radial expansion, a plastic radial constriction of the retractor (2) contrary to the radial expansion of the retractor (2), in that the at least one extractor (6), via its two legs (18, 18', 19, 19'), cooperates with the at least one plate section (5) for the introduction of a torsional moment in order to effect the radial constriction of the retractor (2) in this way.

2. The surgical retractor system (1) according to claim 1, **characterized in that** the extractor (6), with its two legs (18, 18', 19, 19'), forcibly causes the plastic radial constriction of the retractor (2) by means of such a torsional moment (22a, 22b) acting on the retractor (2) so that buckling of the retractor wall in the longitudinal direction of the retractor is brought about.

3. The surgical retractor system (1) according to claim 1 or claim 2,
**characterized in that** a second extractor (6`), via its two legs (18, 18', 19, 19'),cooperates with a second plate section (5') of the same retractor (2) in such a manner that two extractors (6, 6'), via their two legs (18, 18', 19, 19'), bring about a radial constriction of the one retractor (2).

4. The surgical retractor system (1) according to claim 3, **characterized in that** the two extractors (6, 6'), via their two legs (18, 18', 19, 19'), forcibly cause the retractor (2) to be wound up on the extractors (6, 6'), in particular on their operating sections, by means of opposite rotary movements.

5. The surgical retractor system (1) according to any of the claims 1 to 4,
**characterized in that** a predetermined breaking point (16) is arranged in the axial direction (7) along the plate section (5, 5') of a segment (3, 3', 3", 3‴) of the retractor (2) in order to bring about buckling of the retractor (2) at the predetermined breaking point (16) when acted upon with the torsional moment (22a, 22b) via the two legs (18, 18', 19, 19') of the extractor (6, 6').

6. The surgical retractor system (1) according to any of the preceding claims,
**characterized in that** at least the expanding section (4) is designed like a stent in order to allow for the plastic deformation.

7. The surgical retractor system (1) according to any of the preceding claims,
**characterized in that** the at least one extractor (6) is based on a blank having a cross-section which is preferably realized as a round, solid profile bar or hollow profile bar whose connecting section is formed by forming work, in particular bending the bar, and whose operating section comprises at least one longitudinal slit manufactured by a chipping production step such as sawing or grinding, or by a stamping or laser step, said longitudinal slit dividing the distal bar end in the area of the operating section in at least two legs which preferably have the same size, whereby the surgical instrument is suitable as a disposable component.

8. The surgical retractor system according to one of the preceding claims, **characterized in that** the extractor (6, 6') is provided for removing a distractor or retractor (2) from an opening, preferably patient-side opening, which is kept open by said distractor/retractor (2), comprising at least the handle section (10, 10') defining a handle's longitudinal axis (21, 21') and the operating section (9, 9') defining a longitudinal effective axis (20, 20'), which are coupled to each other via a connecting section (22); and the connecting section (22) arranges the handle section (21, 21') and the operating section (9, 9') relative to each other such that the handle's longitudinal axis (21, 21') and the longitudinal effective axis (20, 20') extend at an angle relative to each other, preferably at an angle of between 60° and 140°, particularly preferred 90° whereby the operating section (9, 9') has the longitudinal slit (11, 11') which is open at its distal end, extends along the longitudinal effective axis (20, 20') and divides the operating section (9, 9') in the two legs (18, 18', 19, 19') which are equal in size and separate in circumferential direction.

9. The surgical retractor system according to claim 8, **characterized in that** the handle section (10, 10') has a substantially circular and a substantially constant cross-section over the entire length along the handle's longitudinal axis (21, 21') and **in that** the operating section has a substantially circular and substantially constant cross-section over the entire length along the longitudinal effective axis (20, 20') as well.

10. The surgical retractor system according to claim 9, **characterized in that** the diameter (13, 13') of the handle section (10, 10') corresponds to the diameter (12, 12') of the operating section (9, 9').

11. The surgical retractor system according to one of the claims 8 to 10,
**characterized in that** the first leg (18, 18') of the operating section (9, 9') is prepared and arranged in such a manner so as to contact the retractor (2) from radially inside and the second leg (19, 19') of the operating section (9, 9') is prepared and arranged in such a manner so as to contact the retractor from radially outside.

12. The surgical retractor system according to any of the claims 1 to 11,
**characterized in that** the surgical retractor system is composed of two angled extractors (6, 6') of identical construction.

13. The surgical retractor system according to any of the claims 1 to 12,
**characterized in that** the connecting section (22, 22') regarding the length of its angled route is small as compared to the length of the operating section (9, 9') and the length of the handle section (10, 10').

14. The surgical retractor system (1) according to any of the claims 1 to 13,
**characterized in that** the surgical retractor system (1) is based on a blank having a cross-section which is preferably realized as a round, solid profile bar or hollow profile bar whose connecting section is formed by forming work, in particular bending the bar, and whose operating section comprises at least one longitudinal slit manufactured by a chipping production step such as sawing or grinding, or by a stamping or laser step, said longitudinal slit dividing the distal bar end in the area of the operating section in at least two legs which preferably are of equal size, whereby the surgical retractor system is suitable as a disposable component.

15. The surgical retractor system according to claim 14when dependent on claim 12, **characterized in that** the two extractors (6,6`) are provided and/or held together in order to apply mutually opposed torsion moments simultaneously into the distractor/retractor (2).

## Revendications

1. Système rétracteur chirurgical (1) doté d'un rétracteur essentiellement tubulaire (2), qui comporte dans la direction axiale (7) un nombre de segments (3, 3', 3 ", 3‴), dans lequel au moins un segment (3) est divisé dans la direction périphérique en au moins une section d'expansion (4) et une section de plaque (5, 5'), qui présentent une flexibilité radiale variable entre elles et sont reliées d'un seul tenant l'une à l'autre de sorte qu'une expansion radiale plastique du rétracteur (2) soit possible, dans lequel
le système rétracteur chirurgical (1) présente au moins un extracteur (6, 6') qui présente une section de préhension (10, 10') et une section active (9, 9') avec une fente longitudinale (11, 11') qui est définie par une première branche (18, 18') et une seconde branche (19, 19') et peut être posée sur la section de plaque (5, 5') de sorte que l'extracteur entoure celle-ci sur sa surface orientée radialement vers l'intérieur et sur sa surface orientée radialement vers l'extérieur de manière quasi-linéaire, de sorte que la première branche (18, 18') entre en contact avec le rétracteur (2) radialement de l'intérieur, tandis que la seconde branche (19, 19') entre en contact avec le rétracteur (2) radialement de l'extérieur, de sorte que, dans le cas d'une expansion radiale, une constriction radiale plastique du rétracteur (2) opposée à l'expansion radiale du rétracteur (2) soit provoquée, en faisant interagir le au moins un extracteur (6) via ses deux branches (18, 18', 19, 19') avec la au moins une section de plaque (5) pour une entrée de couple de torsion, afin de produire ainsi la constriction radiale du rétracteur (2).

2. Système rétracteur chirurgical (1) selon la revendication 1, **caractérisé en ce que** l'extracteur (6) avec ses deux branches (18, 18', 19, 19') impose la constriction radiale plastique du rétracteur (2) au moyen d'un couple de torsion (22a, 22b) agissant sur le rétracteur (2), de sorte qu'un flambage de la paroi de rétracteur soit engendré dans la direction longitudinale du rétracteur.

3. Système rétracteur chirurgical (1) selon la revendication 1 ou la revendication 2, **caractérisé en ce qu'**un second extracteur (6') coopère via ses deux branches (18, 18', 19, 19') avec une seconde section de plaque (5') du même rétracteur (2), de sorte que deux extracteurs (6, 6') entraînent via leurs branches (18, 18', 19, 19') une constriction radiale dudit rétracteur (2).

4. Système rétracteur chirurgical (1) selon la revendication 3, **caractérisé en ce que** les deux extracteurs (6, 6') via leurs deux branches (18, 18', 19, 19') forcent un enroulement du rétracteur (2) sur les extracteurs (6, 6'), en particulier de leurs sections actives au moyen d'un mouvement rotatif mutuellement opposé.

5. Système rétracteur chirurgical (1) selon l'une quelconque des revendications 1 à 4, **caractérisé en ce qu'**un point de rupture théorique (16) est agencé dans la direction axiale (7) le long de la section de plaque (5, 5') d'un segment (3, 3', 3", 3‴) du rétracteur (2) pour entraîner un flambage du rétracteur (2) au niveau du point de rupture théorique (16) lorsque le couple de torsion (22a, 22b) est appliqué sur les deux branches (18, 18', 19, 19') de l'extracteur (6, 6').

6. Système rétracteur chirurgical (1) selon l'une quelconque des revendications précédentes, **caractérisé en ce qu'**au moins la section d'expansion (4) est conçue en forme de stent pour permettre la déformation plastique.

7. Système rétracteur chirurgical (1) selon l'une quelconque des revendications précédentes, **caractérisé en ce que** le au moins un extracteur (6) sous forme de pièce brute repose sur une barre profilée pleine ou creuse, de préférence ronde, dans la section transversale, dont la section de liaison est conçue par formage, en particulier par pliage, de la barre et dont la section de fonctionnement présente au moins une fente longitudinale produite par une étape d'usinage par enlèvement de copeaux, telle que le sciage ou le meulage, ou par une étape d'estampage ou laser, qui divise l'extrémité de barre distale dans la zone de la section de fonctionnement en au moins deux branches préférentiellement de même dimensions, ce qui fait de l'instrument chirurgical un composant unidirectionnel approprié.

8. Système rétracteur chirurgical (1) selon l'une quelconque des revendications précédentes, **caractérisé en ce que** l'extracteur (6, 6') est prévu pour détacher un distracteur ou rétracteur (2) d'un orifice maintenu ouvert par le distracteur/rétracteur (2) de préférence côté patient, avec au moins la section de préhension (10, 10') définissant un axe longitudinal de préhension (21, 21') et au moins la section active (9, 9') définissant un axe longitudinal actif (20, 20') qui sont couplées l'une à l'autre par une section de liaison (22) ; et la section de liaison (22) dispose la section de préhension (21, 21') et la section active (9, 9') l'une par rapport à l'autre de sorte que l'axe longitudinal de préhension (21, 21') et l'axe longitudinal actif (20, 20') s'étendent coudés l'un par rapport à l'autre, de préférence selon un angle compris entre 60° et 140°, le plus préférentiellement 90°, dans lequel la section active (9, 9') présente la fente longitudinale (11, 11') ouverte au niveau de son extrémité distale, qui s'étend le long de l'axe longitudinal actif (20, 20') et divise la section active (9, 9') en deux branches (18, 18', 19, 19') de même dimensions séparées sur la périphérie.

9. Système rétracteur chirurgical (1) selon la revendication 8, **caractérisé en ce que** la section de préhension (10, 10') présente sur toute la longueur le long de l'axe longitudinal de préhension (21, 21') une section transversale essentiellement circulaire et essentiellement constante et **en ce que** la section active présente également sur toute la longueur le long de l'axe longitudinal actif (20, 20') une section transversale essentiellement circulaire et essentiellement constante.

10. Système rétracteur chirurgical (1) selon la revendication 9, **caractérisé en ce que** le diamètre (13, 13') de la section de préhension (10, 10') correspond au diamètre (12, 12') de la section active (9, 9').

11. Système rétracteur chirurgical (1) selon l'une quelconque des revendications 8 à 10, **caractérisé en ce que** la première branche (18, 18') de la section active (9, 9') est préparée et agencée de manière à entrer en contact avec le rétracteur (2) radialement de l'intérieur, et la seconde branche (19, 19') de la section active (9, 9') est préparée et agencée pour entrer en contact avec le rétracteur radialement de l'extérieur.

12. Système rétracteur chirurgical (1) selon l'une quelconque des revendications 1 à 11, **caractérisé en ce que** le système rétracteur chirurgical se compose de deux extracteurs coudés de même construction (6, 6').

13. Système rétracteur chirurgical (1) selon l'une quelconque des revendications 1 à 12, **caractérisé en ce que** la section de liaison (22, 22') est petite du point de vue de la longueur de son tracé coudé par rapport à la longueur de la section active (9, 9') et à la longueur de la section de préhension (10, 10').

14. Système rétracteur chirurgical (1) selon l'une quelconque des revendications 1 à 13, **caractérisé en ce que** le système rétracteur (1) sous forme de pièce brute repose sur une barre profilée pleine ou creuse de préférence ronde dans la section transversale, dont la section de liaison est conçue par formage, en particulier par pliage, de la barre et dont la section active présente au moins une fente longitudinale produite par une étape d'usinage par enlèvement de copeaux, telle que le sciage ou le meulage, ou par une étape d'estampage ou laser, qui divise l'extrémité de barre distale dans la zone de la section active en au moins deux branches préférentiellement de même dimensions, ce qui fait de l'instrument chirurgical un composant unidirectionnel approprié.

15. Système rétracteur chirurgical selon la revendication 14, si dépendant de la revendication 12, **caractérisé en ce que** les deux extracteurs (6, 6') sont prévus et/ou sont maintenus l'un contre l'autre de manière à entrer des couples de torsion opposés simultanément dans le distracteur/rétracteur (2).
